# FASCICULE DE BREVET EUROPEEN

(11) **EP 0 707 026 B1**
(45) Date de publication et mention de la délivrance du brevet: **02.02.2000**
(21) Numéro de dépôt: 95401798.4
(22) Date de dépôt: 31.07.1995
(51) Int. Cl.: C08G 69/10, C08G 73/06, C11D 3/37

(54) **Procédé de préparation de polycondensats d'aminoacides et de leur hydrolysats polypeptidiques biodégradables, leur utilisation dans les compositions détergentes**
Verfahren der Herstellung von Aminosäure-Polykondensaten und deren bioabbaubarer Polypeptid-Hydrolysate, ihre Verwendung in Detergens-zusammensetzungen
Preparation of polycondensates of aminoacids and of their biodegradable polypeptidic hydrolysates, their use in detergent compositions

(30) Priorité: 12.10.1994 FR 9412150
(43) Date de publication de la demande: 17.04.1996
(73) Titulaire: RHODIA CHIMIE, 92408 Courbevoie Cédex (FR)
(72) Inventeur: Lepage, Jean-Luc, F-69340 Francheville (FR)
(74) Mandataire: Fabre, Madeleine-France

(56) Documents cités:
- WO-A-92/14753
- WO-A-93/04108
- FR-A- 2 059 475
- FR-A- 2 665 166

## Description

La présente invention a pour objet un procédé de préparation de polycondensats d'aminoacides ou de leurs hydrolysats polypeptidiques biodégradables par polycondensation thermique d'aminoacides en milieu pulvérulent en présence de faibles quantités d'acide phosphorique, d'anhydride phosphorique ou d'acide polyphosphorique, puis éventuellement hydrolyse.

Il est connu de préparer de l'acide polyanhydroaspartique par polycondensation thermique de l'acide aspartique en présence d'acide phosphorique, d'anhydride phosporique ou d'acide polyphosphorique.
Il a été proposé d'opérer sous pression atmosphérique pour obtenir des polysuccinimides de masse molaire limitée ou sous pression réduite pour obtenir des polysuccinimides de masse molaire élevée.
S.W.FOX, K. HARADA (A laboratory Manual of Analytical Methods of Protein Chemistry, including Polypeptides - Pergamon Press, Elmsford, N.Y. - 1966 - p. 129), enseigne que sous pression atmosphérique, en présence d'H₃PO₄, les rendements optimum sont obtenus à 180°C avec un rapport molaire H₃PO₄ / acide aspartique de 3/4 ; dans le cas d'un mélange acide aspartique / acide glutamique selon un rapport molaire 2/1, les meilleurs résultats sont obtenus avec un rapport molaire H₃PO₄ / acide aspartique + acide glutamique de 2/3.
S.W.FOX et K. HARADA proposent également dans US-A-3,052,655, de polycondenser à pression atmosphérique à une température de 150-210°C un mélange acide aspartique / acide glutamique selon un rapport molaire 1/1 en présence d'une quantité équimolaire de H₃PO₄.
La demande de brevet FR-A-2 059 475 décrit la condensation sous pression réduite, en couche mince, de l'acide aspartique à une température de 170-200°C, en présence de H₃PO₄, selon un rapport en poids H₃PO₄ à 85% / acide aspartique de 0,5.
P. NERI, G. ANTONI, F. BENVENUTI, F. COCOLA et G. GAZZEI (Journal of Medicinal Chemistry, 1973, Vol. 16, No. 8) ont étudié l'influence du rapport molaire H₃PO₄ / acide aspartique sur la viscosité du polysuccinimide obtenu par polyconsation à 180°C sous pression réduite en couche fine, le rapport molaire H₃PO₄ / acide aspartique variant de 0,4/1 à 2/1.
La demande de brevet DD-A-262 665 décrit la polycondensation sous vide à 160-200°C de l'acide aspartique en présence d'acide polyphosphorique, avec un rapport acide polyphosphorique / acide aspartique de 1/3 à 2/1.
Il a été proposé dans FR-A-2 665 166 de préparer des polysuccinimides de poids moléculaires très élevés, par polycondensation sous vide à 100-250°C de l'acide aspartique en présence d'acide phosphorique, d'anhydride phosphorique ou d'acide polyphosphorique, selon un rapport pondéral acide phosphorique, anhydride phosporique ou acide polyphosphorique / acide aspartique de 0,1/1 à 2/1, de préférence 0,3/1 à 1/1. L'opération se déroule en deux étapes, la première consistant à préparer un polysuccinimide ayant un poids moléculaire de 10 000 à 100 000, la deuxième consistant à fragmenter mécaniquement le milieu réactionnel dur et compact et à poursuivre la polycondensation.

Tous ces procédés présentent l'inconvénient de mettre en oeuvre une forte quantité d'acide phosphorique ou polyphosphorique (à éliminer postérieurement), avec passage du milieu réactionnel par une phase plus ou moins visqueuse, avec un moussage important (nécessité de casser les mousses), puis solidification du milieu en fin de polycondensation (broyage final obligatoire). De tels procédés sont difficilement réalisables industriellement.

La demanderesse a trouvé un mode opératoire permettant de mettre en oeuvre des quantités d'acide phosphorique, d'anhydride phosphorique ou d'acide polyphosphorique nettement inférieures à 40 % molaire sans formation de mousses et sans prise en masse du milieu réactionnel.

Selon l'invention il s'agit d'un procédé de préparation de polycondensats polyimides d'aminoacides ou de leurs hydrolysats polypeptidiques, par polycondensation thermique en masse d'aminoacides en présence d'acide phosphorique, d'anhydride phosphorique ou d'acide polyphosphorique comme catalyseur de polycondensation, puis éventuellement hydrolyse, ledit procédé étant caractérisé en ce que l'opération de polycondensation est réalisée en milieu pulvérulent contenant pour une molecule d'aminoacide, de 0,005 à 0,25 mole, de préférence de 0,01 à 0,18 mole, de catalyseur uniformément réparti dans ledit milieu, ledit milieu étant susceptible d'être obtenu par :
- empâtage d'un mélange d'aminoacide et d'acide phosphorique ou polyphosphorique par de l'eau, élimination de l'eau par évaporation à pression atmosphérique ou de préférence sous vide , puis broyage de la masse obtenue
- solubilisation dans l'eau, de préférence à chaud, de l'aminoacide et de l'acide phosphorique ou polyphosphorique, puis atomisation/séchage de la solution
- atomisation/séchage d'une suspension d'aminoacide dans une solution aqueuse d'acide phosphorique ou polyphosphorique
- atomisation/séchage d'une solution aqueuse d'acide phosphorique ou polyphosphorique sur un lit fluidisé d'aminoacide
- ou cobroyage ou micronisation de l'anhydride phosphorique et de l'aminoacide.

Dans la définition de la mole de catalyseur, on prendra comme entité élémentaire la molecule dans le cas de l'acide phosphorique, l'atome de P dans le cas de l'anhydride phosphorique ou de l'acide polyphosphorique (un atome de P étant équivalent à une molécule d'acide phosphorique).

Dans la définition de la mole d'aminoacide, on prendra comme entité élémentaire la molécule.

Parmi les aminoacides pouvant être mis en oeuvre pour réaliser le procédé de l'invention, on peut citer l'acide aspartique ou l'acide glutamique pris seuls ou en mélange entre eux dans des proportions quelconques ou en mélange avec un aminoacide autre (par exemple jusqu'à 15% en poids, de préférence moins de 5% en poids de glycine, alanine, valine, leucine, isoleucine, phenylalanine, méthionine, histidine, proline, lysine, sérine, thréonine, cysteine, ...)
L'aminoacide préféré est l'acide aspartique.

L'opération de polycondensation thermique peut être réalisée à une température de 150 à 220°C, de préférence à une température de 180 à 200°C.
Ladite opération peut être réalisée à une température choisie ou suivant un profil de température préétabli.

Cette opération de polycondensation thermique peut être réalisée sous vide (de préférence supérieur à 1mbar), à pression atmosphérique ou éventuellement sous pression (par exemple jusqu'à 20 bar), suivant la masse molaire désirée.

Tout moyen d'introduction du catalyseur aux taux sus-mentionnés permettant d'obtenir un mélange pulvérulent, dans lequel ledit catalyseur est uniformément réparti, peut être mis en oeuvre.

Un mode particulièrement intéressant consiste à utiliser de l'acide phosphorique ou polyphosphorique comme catalyseur, à introduire l'acide phosphorique ou polyphosphorique dans l'aminoacide, à homogéneiser le mélange par empâtage à l'aide d'une quantité d'eau suffisante pour obtenir un milieu pâteux homogène (de préférence de 0,4 à 1 partie en poids d'eau pour 1 partie en poids d'aminoacide), à éliminer l'eau excédentaire du mélange par séchage à pression atmosphérique ou de préférence sous vide (généralement inférieur à 10000 Pa), à broyer la masse obtenue pour obtenir un milieu pulvérulent, puis à réaliser l'opération de polycondensation.
On entend par "eau excédentaire", l'eau qui n'est pas en équilibre avec l'acide phosphorique ou polyphosphorique à la température et à la pression de séchage.

Le polyimide obtenu selon le procédé de l'invention peut être, si nécessaire, séparé, filtré, purifié et séché.
Le catalyseur peut être si désiré séparé du polyimide par lavage à l'eau ou à l'aide d'un solvant du catalyseur et non solvant du polyimide.
Le polyimide peut être purifié par solubilisation à l'aide d'un solvant polaire aprotique (diméthylformamide, formamide, diméthylsulfoxyde...) puis reprécipitation à l'aide d'un composé non solvant dudit polyimide (eau, éther, éthanol, acétone ...)

Le polyimide obtenu , séparé ou non, peut ensuite, être hydrolysé de préférence par addition d'un agent basique (base alcaline, alcalino-terreuse, carbonate alcalin ou alcalino-terreux ...) en présence d'eau si nécessaire, en milieu homogène ou biphasique ; dans le cas du polysuccinimide dérivé de l'acide aspartique, l'hydrolysat ainsi obtenu est constitué de polyaspartate (de sodium par exemple).

La forme acide de l'hydrolysat peut être obtenue par exemple par neutralisation du sel obtenu ci-dessus par hydrolyse alcaline, à l'aide d'un acide organique ou minéral (HCI notamment) ; dans le cas du polysuccinimide dérivé de l'acide aspartique, l'hydrolysat ainsi obtenu est constitué d'acide polyaspartique.

On entend par "hydrolysat", selon l'invention, le produit obtenu par hydrolyse (par action de l'eau) partielle ou totale du polyimide formé ; cette hydrolyse conduit par ouverture des cycles imides, à la formation de fonctions amides d'une part et de fonctions acides carboxyliques ou sels d'acides carboxyliques d'autre part.

Les produits obtenus selon le procédé de la présente invention peuvent être utilisés notamment comme agent ou coagent polypeptidique biodégradable améliorant les performances des agents de surface (agent ou coagent dénommé "builder" ou "cobuilder") dans les compositions détergentes (lessives lave-linge, lessives lave-vaisselle ou tout autre autre produit de lavage à usage ménager).

Les exemples suivants sont donnés à titre indicatif.
L'indice de viscosité (IV) donné dans ces exemples est mesuré à l'aide d'un viscosimètre capillaire SCHOTT AVS 350 à partir :
- soit d'une quantité de polysuccimide (PSI) mis en solution dans de la soude 0,5N de manière à avoir une concentration de 0,002 g/ml à une température de 25°C (indice de viscosité IV1)
- soit d'une quantité de polysuccimide (PSI) mis en solution dans le diméthylformamide à une concentration de 0,005 g/ml à une température de 25°C (indice de viscosité IV2)
Dans ces exemples la biodégradabilité des hydrolysats est mesurée selon la norme AFNOR T90-312 (en conformité avec la norme internationale ISO 7827 du 15 octobre 1984).
Le test est réalisé à partir :
- d'un inoculum obtenu par filtration d'eau d'entrée de la station urbaine de Saint Germain au Mont d'Or (Rhône), tel quel ou après adaptation
- d'un milieu d'essai contenant 4x10⁵ bactéries/ml
- d'une quantité de produit à tester telle que le milieu d'essai contienne une concentration en carbone organique de de l'ordre de 40ml/g.
Le taux de biodégradabilité est mesuré en fonction du temps dans les conditions de rejet en eau de rivière.
Pour cette mesure les échantillons testés ont été obtenus par hydrolyse par une solution diluée de soude des polysuccinimides préparés, jusqu'à obtenir une solution à 6% environ en polyaspartate de sodium, de pH de l'ordre de 9 à 11.
Le niveau de biodégradabilité est caractérisé par les deux paramètres suivants :
. le taux maximum de biodégradation (TMB)
. le temps nécessaire pour passer d'un taux de biodégradation de 10% à un taux de 90% du taux maximum de biodégradation (t 10-90).
Mesure de la coloration selon la méthode HUNTER Lab
La coloration des poudres de polysuccinimides ou de leurs hydrolysats, est déterminée par mesure sur un spectro-colorimètre ACS SPECTRO-SENSOR II®.
- Conditions de mesure :
   . illuminant : D 65
   . angle d'observation : 2°
Les 3 valeurs mesurées sont
. L : Luminance qui varie entre 0 (noir) et 100 (blanc)
. a : Coloration rouge (a positif) / vert (a négatif)
. b : Coloration jaune (b positif) / bleu (b négatif)
Ces valeurs sont calculées, à partir des mesures selon les formules
L = 100 (Y/Yo)^{1/2} a = Ka (X/Xo-Y/Yo) / (Y/Yo)^{1/2} b = Kb (Y/Yo-Z/Zo) / (Y/Yo)^{1/2}
X, Y, Z : valeurs tristimuli correspondant aux 3 couleurs primaires obtenues avec l'échantillon ;
Xo, Yo, Zo : valeurs tristimuli du diffuseur parfait de la source utilisée ;
Ka, Kb : coefficients pour la source utilisée.

### Exemple 1

On prépare un mélange réactionnel pulvérulent par :
- incorporation de 20g d'acide orthophosphorique à 85% dans 400g d'acide L-aspartique, avec homogéneisaton du milieu par empâtage à l'aide de 320g d'eau, sur un plateau émaillé
- puis évaporation de l'eau et séchage dans une étuve à vide pendant 16 heures à 40°C sous 6000 Pa. et 21 heures à 80°C sous 6000 Pa.
- et broyage des 416,7g de mélange obtenu, à l'aide d'un broyeur à couteaux. On introduit 51,8g de ce mélange pulvérulent dans un ballon d'évaporateur rotatif de 250ml préchauffé à 200°C (température du bain d'huile). La vitesse de rotation du ballon est de 20 tours/mn.
On récupère après polycondensation pendant 4 heures à 200°C à pression atmosphérique, sans lavage à l'eau, 42,0 g de produit, ce qui correspond à un rendement en acide polyanhydroaspartique (polysuccinimide PSI) de 72,0% (confirmation par dosage par potentiométrique).
Aucun mottage de la masse réactionnelle n'a été constaté au cours de l'opération de polycondensation.
Le produit présente un indice de viscosité IV1 =13,6ml/g.et une coloration HUNTER
L =91,2
a =1,3
b =10,2

### Exemple 2

On prépare un mélange réactionnel pulvérulent par :
- incorporation de 80g d'acide orthophosphorique à 85% dans 800g d'acide L aspartique, avec homogéneisaton du milieu par empâtage à l'aide de 640g d'eau, sur un plateau émaillé.
- puis évaporation de l'eau et séchage dans une étuve à vide pendant 20 heures à 40°C sous 6000 Pa., 25 heures à 80°C sous 6000 Pa. et 2 heures à 80°C sous 650 Pa.
- et broyage des 867,9g de mélange obtenu, à l'aide d'un broyeur à couteaux.
On introduit 53,4g de ce mélange pulvérulent dans un ballon d'évaporateur rotatif de 250ml préchauffé à 200°C (température du bain d'huile). La vitesse de rotation du ballon est de 20 tours/mn.
On récupère après polycondensation pendant 4 heures à 200°C à pression atmosphérique, sans lavage à l'eau, 41,0 g de produit, ce qui correspond à un rendement en acide polyanhydroaspartique (polysuccinimide PSI) de 92,0% (confirmation par dosage par potentiométrique).
Aucun mottage de la masse réactionnelle n'a été constaté au cours de l'opération de polycondensation.
Le produit présente un indice de viscosité IV1 =15,7ml/g.et une coloration HUNTER
L =90,7
a =-0,4
b =10,8
Ce polysuccinimide est hydrolysé comme ci-dessus indiqué ; la biodégradabilité de l'hydrolysat est la suivante :
TMB : 97%
t 10-90 :7 jours

### Exemple 3

On prépare un mélange réactionnel pulvérulent par :
- incorporation de 7,5g d'acide orthophosphorique à 85% dans 50g d'acide L aspartique, avec homogeneisaton du milieu par empâtage à l'aide de 21g d'eau dans un évaporateur rotatif muni d'un ballon de 250ml.
- puis évaporation de l'eau et séchage sous vide dans cet évaporateur rotatif à 80°C, pendant 35 minutes sous 4600 Pa. et 4 heures sous 1300 Pa.
- et broyage des 56,3g de mélange obtenu, à l'aide d'un mortier.
On introduit 55,2g de ce mélange pulvérulent dans un ballon d'évaporateur rotatif de 250ml préchauffé à 200°C (température du bain d'huile). La vitesse de rotation du ballon est de 20 tours/mn.
On récupère après polycondensation pendant 4 heures à 200°C à pression atmosphérique, sans lavage à l'eau, 42,3 g de produit, ce qui correspond à un rendement en acide polyanhydroaspartique (polysuccinimide PSI) de 100% (confirmation par dosage par potentiométrique).
On constate un mottage du milieu en début de polycondensation, éliminé par grattage de la masse réactionnelle ; l'essai se déroule ensuite en milieu pulvérulent sans aucune prise en masse.
Le produit présente un indice de viscosité IV1 =14,7ml/g.et une coloration HUNTER
L =90,4
a =-0,7
b =11,1

### Exemple 4

On prépare un mélange réactionnel pulvérulent par :
- incorporation de 2,5g d'acide orthophosphorique à 85% dans 50g d'acide L aspartique, avec homogeneisaton du milieu par empâtage à l'aide de 20g d'eau dans un évaporateur rotatif muni d'un ballon de 250ml.
- puis évaporation de l'eau et séchage sous vide dans cet évaporateur rotatif à 80°C, pendant 20 minutes sous 6000Pa. et 4 heures sous 1300 Pa.
- et broyage des 52,2g de mélange obtenu, à l'aide d'un mortier.
On introduit 51,9g de ce mélange pulvérulent dans un ballon d'évaporateur rotatif de 1 litre préchauffé à 200°C (température du bain d'huile). La vitesse de rotation du ballon est de 20 tours/mn.
On récupère après polycondensation pendant 6 heures à 180°C à pression atmosphérique, sans lavage à l'eau, 44,0 g de produit, ce qui correspond à un rendement en acide polyanhydroaspartique (polysuccinimide PSI) de 60% (confirmation par dosage par potentiométrique).
On ne constate pas de mottage pendant la polycondensation.
Le produit présente un indice de viscosté IV2 de 12,9 et une coloration HUNTER
L =93,3
a =0,3
b =8,2

### Exemple 5

On prépare un mélange réactionnel pulvérulent par :
- incorporation de 5,0g d'acide orthophosphorique à 85% dans 50g d'acide L aspartique, avec homogeneisaton du milieu par empâtage à l'aide de 20g d'eau, dans un évaporateur rotatif muni d'un ballon de 250ml.
- puis évaporation de l'eau et séchage sous vide dans cet évaporateur rotatif à 80°C, pendant 1h 20mn sous 8000 Pa. et 2h 30mn sous 1300 Pa.
- et broyage des 54,3g de mélange obtenu, à l'aide d'un mortier.
On introduit 53,8g de ce mélange pulvérulent dans un ballon d'évaporateur rotatif de 250ml préchauffé à 200°C (température du bain). La vitesse de rotation du ballon est de 20 tours/mn.
On récupère après polycondensation pendant 6 heures à 180°C à pression atmosphérique, sans lavage à l'eau, 42,0 g de produit, ce qui correspond à un rendement en acide polyanhydroaspartique (polysuccinimide PSI) de 91% (confirmation par dosage par potentiométrique).
On ne constate pas de mottage pendant la polycondensation.
Le produit présente un indice de viscosté IV1 de 13,9 et une coloration HUNTER
L =94,1
a =-0,9
b =7,2
Ce polysuccinimide est hydrolysé comme ci-dessus indiqué ; la biodégradabilité de l'hydrolysat est la suivante :
TMB : 94%
t 10-90 : 5 jours

### Exemple 6

On prépare un mélange réactionnel pulvérulent par :
- incorporation de 7,5g d'acide orthophosphorique à 85% dans 50g d'acide L aspartique, avec homogeneisaton du milieu par empâtage à l'aide de 20,3g d'eau, dans un évaporateur rotatif muni d'un ballon de 250ml.
- puis évaporation de l'eau sous vide dans cet évaporateur rotatif à 80°C, pendant 30mn sous 4600 Pa. et 4h sous 650 Pa.
- et broyage des 56,6g de mélange obtenu, à l'aide d'un mortier.
On introduit 55,4g de ce mélange pulvérulent dans un ballon d'évaporateur rotatif de 250ml préchauffé à 200°C (température du bain d'huile). La vitesse de rotation du ballon est de 20 tours/mn.
On récupère après polycondensation pendant 6 heures à 180°C à pression atmosphérique, sans lavage à l'eau, 42,9g de produit, ce qui correspond à un rendement en acide polyanhydroaspartique (polysuccinimide PSI) de 97% (confirmation par dosage par potentiométrique).
On constate un mottage du milieu en début de polycondensation, éliminé par grattage de la masse réactionnelle ; l'essai se déroule ensuite en milieu pulvérulent sans aucune prise en masse.
Le produit présente un indice de viscosté IV1 de 14,7 et une coloration HUNTER
L =92,8
a =-0,9
b =8,8

### Exemple 7

On prépare un mélange réactionnel pulvérulent par :
- incorporation de 20g d'acide orthophosphorique à 85% dans 400g d'acide L aspartique, avec homogeneisaton du milieu par empâtage à l'aide de 320g d'eau, dans un évaporateur rotatif muni d'un ballon de 3 litres
- puis évaporation de l'eau sous vide dans cet évaporateur rotatif à 80°C, pendant 1 heure sous 2600 Pa. et 4 h 15mn sous 1300 Pa., puis séchage complémentaire dans une étuve à vide pendant 65 heures à 80°C et sous 6600 Pa.
- et broyage des 413g de mélange obtenu, à l'aide d'un mortier.
On introduit 50g de ce mélange pulvérulent dans un ballon d'évaporateur rotatif de 250ml préchauffé à 200°C (température du bain d'huile). La vitesse de rotation du ballon est de 20 tours/mn.
On récupère après polycondensation pendant 6 heures à 180°C sous vide (1300 Pa.), sans lavage à l'eau, 39,7 g de produit, ce qui correspond à un rendement en acide polyanhydroaspartique (polysuccinimide PSI) de 79% (confirmation par dosage par potentiométrique).
On ne constate pas de mottage pendant la polycondensation.
Le produit présente un indice de viscosté IV1 de de 33,6 et une coloration HUNTER
L =89,7
a =0,8
b =13,5

### Exemple 8

On prépare un mélange réactionnel pulvérulent par :
- incorporation de 7,8g d'acide orthophosphorique à 85% dans 50g d'acide L aspartique, avec homogeneisaton du milieu par empâtage à l'aide de 20g d'eau, dans un évaporateur rotatif muni d'un ballon de 250ml.
- puis évaporation de l'eau sous vide dans cet évaporateur rotatif à 80°C, pendant 35mn sous 4600 Pa. et 4h sous 1300 Pa.
- et broyage des 56,3g de mélange obtenu, à l'aide d'un mortier.
On introduit 55,5g de ce mélange pulvérulent dans un ballon d'évaporateur rotatif de 250ml préchauffé à 200°C (température du bain). La vitesse de rotation du ballon est de 26 tours/mn.
On récupère après polycondensation pendant 3 heures à 200°C sous vide (1300 Pa.), sans lavage à l'eau, 41,8 g de produit, ce qui correspond à un rendement en acide polyanhydroaspartique (polysuccinimide PSI) de 100% (confirmation par dosage par potentiométrique).
On constate un mottage du milieu en début de polycondensation, éliminé par grattage de la masse réactionnelle ; l'essai se déroule ensuite en milieu pulvérulent sans aucune prise en masse.
Le produit présente un indice de viscosité IV2 =16,6ml/g et une coloration HUNTER
L =89,7
a =-0,5
b =14,3

### Exemple 9 comparatif

Dans un ballon d'évaporateur rotatif de 2 litres, on introduit
- 50g d'acide L aspartique
- 25g d'acide phosphorique à 85%
On chauffe les réactifs à 180°C (température du bain d'huile) à pression atmosphérique pendant 4 heures, la vitesse de rotation du ballon étant de 90 tours/mn (vitesse nécessaire pour éviter le moussage du milieu réactionnel ; à faible vitesse de rotation, par exemple 20 tours/mn, la mousse déborde du ballon).
On constate au cours de l'opération de polycondensation, la formation d'une phase visqueuse intermédiaire, une légère expansion, puis la prise en masse du milieu réactionnel.
La masse réactionnelle obtenue est broyée au mortier, ensuite lavée par 3 x 1,8 litre d'eau, puis séchée pendant 31 heures à 60°C sous 8000 Pa.
On récupère 34,85g de PSI (soit un rendement de 95,6%).
Le produit présente un indice de viscosité IV2 égal à 11,3ml/g et une coloration HUNTER
L =92,0
a =-0,3
b =6,9

### Exemple 10 comparatif

Dans un ballon d'évaporateur rotatif de 2 litres, on introduit
- 50g d'acide L aspartique
- 25g d'acide phosphorique à 85%
On chauffe les réactifs à 180°C (température du bain d'huile) sous 1300 Pa. pendant 2h 35mn, la vitesse de rotation du ballon étant de 90 tours/mn (vitesse nécessaire pour éviter le moussage du milieu réactionnel).
On constate au cours de l'opération de polycondensation, la formation d'une phase visqueuse intermédiaire, puis la prise en masse du milieu réactionnel.
La masse réactionnelle obtenue est broyée au mortier, ensuite lavée par 3 x 1,6 litre d'eau, puis séchée pendant 51 heures à 60°C sous 8000 Pa.
On récupère 37,2g de PSI (soit un rendement de 100%).
Le produit présente un indice de viscosité IV2 égal à 32,5ml/g et une coloration HUNTER
L =88,8
a =-0,0
b =11,6

## Revendications

1. Procédé de préparation de polycondensats potyimides d'aminoacides ou de leurs hydrolysats polypeptidiques, par polycondensation thermique en masse d'aminoacides en présence d'acide phosphorique, d'anhydride phosphorique ou d'acide polyphosphorique comme catalyseur de polycondensation, puis éventuellement hydrolyse, ledit procédé étant caractérisé en ce que l'opération de polycondensation est réalisée en milieu pulvérulent contenant pour une molecule d'aminoacide, de 0,005 à 0,25 mole de catalyseur uniformément réparti dans ledit milieu, ledit milieu étant susceptible d'être obtenu par :
- empâtage d'un mélange d'aminoacide et d'acide phosphorique ou polyphosphorique par de l'eau, élimination de l'eau par évaporation à pression atmosphérique ou de préférence sous vide , puis broyage de la masse obtenue
- solubilisation dans l'eau, de préférence à chaud, de l'aminoacide et de l'acide phosphorique ou polyphosphorique , puis atomisation/séchage de la solution
- atomisation/séchage d'une suspension d'aminoacide dans une solution aqueuse d'acide phosphorique ou polyphosphorique
- atomisation/séchage d'une solution aqueuse d'acide phosphorique ou polyphosphorique sur un lit fluidisé d'aminoacide
- ou cobroyage ou micronisation de l'anhydride phosphorique et de l'aminoacide.

2. Procédé selon la revendication 1), caractérisé en ce que l'opération de polycondensation est réalisée en milieu pulvérulent contenant pour une molecule d'aminoacide, de 0,01 à 0,18 mole, de catalyseur.

3. Procédé selon la revendication 1) ou 2), caractérisé en ce que l'aminoacide mis en oeuvre est l'acide aspartique ou l'acide glutamique pris seuls ou en mélange entre eux dans des proportions quelconques ou en mélange avec un aminoacide autre.

4. Procédé selon l'une quelconque des revendications 1) à 3), caractérisé en ce que l'opération de polycondensation thermique est réalisée à une température de 150 à 220°C.

5. Procédé selon la revendication 4), caractérisé en ce que l'opération de polycondensation thermique est réalisée à une température de 180 à 200°C.

6. Procédé selon l'une quelconque des revendications 1) à 5), caractérisé en ce que le catalyseur utilisé est de l'acide phosphorique ou polyphosphorique, et ce que le milieu réactionnel pulvérulent soumis à l'opération de polycondensation et renfermant le catalyseur réparti d'une manière uniforme, est obtenu par introduction de l'acide phosphorique ou polyphosphorique dans l'aminoacide, homogéneisation du mélange par empâtage à l'aide d'une quantité d'eau suffisante pour obtenir un milieu pâteux homogène, élimination de l'eau excédentaire du mélange par séchage à pression atmosphérique ou de préférence sous vide, et broyage de la masse obtenue pour obtenir un milieu pulvérulent

7. Procédé selon la revendication 6), caractérisé en ce que l'homogéneisation du mélange par empâtage est réalisée à l'aide de 0,4 à 1 partie en poids d'eau pour 1 partie en poids d'aminoacide.

8. Procédé selon la revendication 6), caractérisé en ce que l'élimination de reau excédentaire du mélange par séchage est réalisée à une pression inférieure à 10000 Pa.

9. Procédé selon l'une quelconque des revendications 1) à 8), caractérisé en ce que le polycondensat polyimide obtenu par thermocondensation est hydrolysé en un sel polypeptidique par addition d'un agent basique, ledit sel étant ensuite éventuellement neutralisé en un acide polypeptidique par addition d'un acide minéral ou organique.

## Claims

1. Process for the preparation of polyimide polycondensates of amino acids or of the polypeptide hydrolysates thereof, by bulk thermal polycondensation of amino acids in the presence of phosphoric acid, phosphorus pentoxide or polyphosphoric acid as polycondensation catalyst, optionally followed by hydrolysis, the said process being characterized in that the polycondensation operation is performed in pulverulent medium containing, per molecule of amino acid, from 0.005 to 0.25 mol of catalyst uniformly distributed in the said medium, it being possible for the said medium to be obtained by
- slurrying of a mixture of amino acid and phosphoric acid or polyphosphoric acid with water, removal of the water by evaporation at atmospheric pressure or, preferably, under vacuum, followed by grinding of the mass obtained
- solubilization in water, preferably hot water, of the amino acid and the phosphoric acid or polyphosphoric acid, followed by spraying/drying of the solution
- spraying/drying of a suspension of amino acid in an aqueous solution of phosphoric acid or polyphosphoric acid
- spraying/drying of an aqueous solution of phosphoric acid or polyphosphoric acid on a fluidized bed of amino acid
- or co-grinding or micronization of the phosphorus pentoxide and the amino acid.

2. Process according to Claim 1, characterized in that the polycondensation operation is performed in pulverulent medium containing from 0.01 to 0.18 mol of catalyst per molecule of amino acid.

3. Process according to Claim 1 or 2, characterized in that the amino acid used is aspartic acid or glutamic acid, taken alone or mixed together in any proportions, or mixed with another amino acid.

4. Process according to any one of Claims 1 to 3, characterized in that the thermal polycondensation operation is performed at a temperature of from 150 to 220°C.

5. Process according to Claim 4, characterized in that the thermal polycondensation operation is performed at a temperature of from 180 to 200°C.

6. Process according to any one of Claims 1 to 5, characterized in that the catalyst used is phosphoric acid or polyphosphoric acid, and in that the pulverulent reaction medium undergoing the polycondensation operation and containing the uniformly distributed catalyst is obtained by introduction of the phosphoric acid or polyphosphoric acid into the amino acid, homogenization of the mixture by slurrying using an amount of water which is sufficient to obtain a homogeneous pasty medium, removal of the excess water from the mixture by drying at atmospheric pressure or, preferably, under vacuum, and grinding of the mass obtained in order to obtain a pulverulent medium.

7. Process according to Claim 6, characterized in that the homogenization of the mixture by slurrying is performed using from 0.4 to 1 part by weight of water per 1 part by weight of amino acid.

8. Process according to Claim 6, characterized in that the removal of the excess water from the mixture by drying is performed at a pressure below 10,000 Pa.

9. Process according to any one of Claims 1 to 8, characterized in that the polyimide polycondensate obtained by thermal condensation is hydrolysed to a polypeptide salt by addition of a basic agent, the said salt then being optionally neutralized to a polypeptidic acid by addition of an inorganic or organic acid.

## Patentansprüche

1. Verfahren zur Herstellung von Aminosäurepolyimid-Polykondensaten oder deren Polypeptid-Hydrolysaten durch thermische Polykondensation von Aminosäuren in Substanz in Gegenwart von Phosphorsäure, Phosphorsäureanhydrid oder Polyphosphorsäure als Polykondensations-Katalysator und gegebenenfalls anschließende Hydrolyse, wobei dieses Verfahren dadurch gekennzeichnet ist, daß der Polykondensationsschritt in einem feinpulverigen Medium durchgeführt wird, das pro mol Aminosäure 0,005 bis 0,25 mol gleichmäßig in diesem Medium verteilten Katalysator enthält und das folgendermaßen erhalten werden kann:
- durch Aufschlämmen eines Gemischs aus Aminosäure und Phosphorsäure beziehungsweise Polyphosphorsäure mit Wasser, Entfernen des Wassers durch Verdampfen bei Atmosphärendruck oder vorzugsweise unter Vakuum und anschließendes Vermahlen der erhaltenen Masse,
- durch Solubilisieren der Aminosäure und der Phosphorsäure beziehungsweise der Polyphosphorsäure in Wasser - vorzugsweise in der Hitze - und anschließendes Vernebeln und Trocknen der Lösung,
- durch Vernebeln und Trocknen einer Aminosäure-Suspension in einer wässerigen Lösung von Phosphorsäure beziehungsweise Polyphosphorsäure,
- durch Vernebeln und Trocknen einer wässerigen Lösung von Phosphorsäure beziehungsweise Polyphosphorsäure über einem Aminosäure-Wirbelschichtbett oder
- durch gemeinsames Vermahlen oder Mikronisieren des Phosphorsäureanhydrids und der Aminosäure.

2. Verfahren nach Anspruch 1, dadurch gekennzeichnet, daß der Polykondensationsschritt mit einem feinpulverigen Medium durchgeführt wird, das pro mol Aminosäure 0,01 bis 0,18 mol Katalsyator enthält.

3. Verfahren nach Anspruch 1 oder 2, dadurch gekennzeichnet, daß die eingesetzte Aminosäure Asparaginsäure, Glutaminsäure, ein Gemisch dieser beiden Aminosäuren in einem beliebigen Mischungsverhältnis oder ein Gemisch einer dieser beiden Aminosäuren mit einer anderen Aminosäure ist.

4. Verfahren nach einem der Ansprüche 1 bis 3, dadurch gekennzeichnet, daß der thermische Polykondensationsschritt bei einer Temperatur von 150 bis 220 °C durchgeführt wird.

5. Verfahren nach Anspruch 4, dadurch gekennzeichnet, daß der thermische Polykondensationsschritt bei einer Temperatur von 180 bis 200 °C durchgeführt wird.

6. Verfahren nach einem der Ansprüche 1 bis 5, dadurch gekennzeichnet, daß der verwendete Katalysator Phosphorsäure oder Polyphosphorsäure ist und daß das dem Polykondensationsschritt unterzogene feinpulverige Reaktionsmedium, in dem gleichmäßig verteilt der Katalysator enthalten ist, erhalten wird, indem die Aminosäure mit Phosphorsäure oder Polyphosphorsäure versetzt wird, das Gemisch durch Aufschlämmen mit einer zum Erhalt eines homogenen, pastösen Mediums ausreichenden Menge an Wasser homogenisiert wird, das überschüssige Wasser des Gemischs durch Trocknen bei Atmosphärendruck oder vorzugsweise unter Vakuum entfernt wird und die erhaltene Masse unter Erhalt eines feinpulverigen Mediums vermahlen wird.

7. Verfahren nach Anspruch 6, dadurch gekennzeichnet, daß das Homogenisieren des Gemischs durch Aufschlämmen mit 0,4 bis 1 Gewichtsteil Wasser pro Gewichtsteil Aminosäure durchgeführt wird.

8. Verfahren nach Anspruch 6, dadurch gekennzeichnet, daß das Entfernen des überschüssigen Wassers durch Trocknen bei einem Druck kleiner 10 000 Pa durchgeführt wird.

9. Verfahren nach einem der Ansprüche 1 bis 8, dadurch gekennzeichnet, daß das durch Thermokondensation erhaltene Polyimid-Polykondensat durch Zugabe einer Base unter Erhalt eines Polypeptidsalzes hydrolysiert wird, welches anschließend gegebenenfalls durch Zugabe einer mineralischen oder organischen Säure unter Erhalt einer Polypeptidsäure neutralisiert wird.
